# EUROPEAN PATENT APPLICATION

(11) **EP 4 549 422 A1**
(43) Date of publication of application: **07.05.2025**
(21) Application number: 24841408.8
(22) Date of filing: 12.07.2024
(51) Int. Cl.: C07C 17/389, B01D 53/04, C07C 19/08, C07C 21/18, C07C 63/307, C07F 1/08

(54) **SEPARATION METHOD**

(30) Priority: 23.08.2023 JP 2023135708
(71) Applicant: Daikin Industries, Ltd., Osaka-shi, Osaka 530-0001 (JP)
(72) Inventor: HAGITA, Satoru, Osaka-Shi, Osaka 530-0001 (JP); HADA, Ryosuke, Osaka-Shi, Osaka 530-0001 (JP); KAGAWA, Michiru, Osaka-Shi, Osaka 530-0001 (JP)
(74) Representative: Hoffmann Eitle
(86) International application number: PCT/JP2024/025306
(87) International publication number: WO 2025/041479

(57) **Abstract**

The present invention relates to a method for separating a fluorocarbon.

## Description

### Technical Field

The present disclosure relates to a separation method.

### Background Art

A known method for separating fluorocarbon includes distilling a mixture of fluorocarbons. Patent Literature 1 describes that a mixture comprising 2,3,3,3-tetrafluoropropene and hexafluoropropene is mixed with an extraction solvent to obtain a mixture for extraction, and that such a mixture for extraction is then distilled to obtain a distillate comprising hexafluoropropene as a main component and a bottom comprising 2,3,3,3-tetrafluoropropene.

### Citation List

### Patent Literature

Patent Literature 1: JP2018-002602 A

### Summary

### Technical Problem

In a distillation method described in Patent Literature 1, the separation of compounds having similar boiling points or azeotropic compounds was found difficult, and disadvantageously, separation of a fluorocarbon, for example, a fluorocarbon which has a double bond or no double bonds, may be insufficient.

The present disclosure is to provide a novel method for separating a fluorocarbon.

### Solution to Problem

The present disclosure comprises the following aspects:
[1] A method for separating a fluorocarbon, comprising:
   separating, from a first mixture comprising a fluorocarbon A having n carbon atoms and one or more double bonds and a fluorocarbon B having m carbon atoms and no double bonds,
   a second mixture in which a composition ratio between the fluorocarbon A and the fluorocarbon B is different from that in the first mixture,
   wherein the separation is performed by bringing the first mixture and an adsorbent comprising a metal-organic framework into contact with each other.
[2] The method according to [1], wherein the first mixture and the adsorbent comprising a metal-organic framework are brought into contact with each other at a temperature of -10°C or higher and 80°C or lower.
[3] The method according to [1] or [2], wherein n is 2 or more and 3 or less.
[4] The method according to any one of [1] to [3], wherein m is 1 or more and 3 or less.
[5] The method according to any one of [1] to [4], wherein the fluorocarbon A comprises R1234yf.
[6] The method according to any one of [1] to [5], wherein the fluorocarbon B comprises one or more selected from R134a, R143, R143a, R32 and R125.
[7] The method according to any one of [1] to [6], wherein the metal-organic framework comprises a metal ion.
[8] The method according to any one of [1] to [7], wherein the metal-organic framework comprises one or more organic ligands, and the organic ligand comprises two or more groups capable of forming a coordinate bond to a metal ion, per molecule.
[9] The method according to any one of [1] to [8], wherein the metal-organic framework comprises a metal ion and one or more organic ligands, and
   the organic ligand comprises two or more groups capable of forming a coordinate bond to the metal ion, per molecule.
[10] The method according to any one of [1] to [9], wherein the metal-organic framework comprises an open metal site provided by a coordinatively unsaturated metal ion.
[11] The method according to any one of [1] to [10], wherein the adsorbent comprises the metal-organic framework further comprises a resin.
[12] The method according to any one of [1] to [11], wherein the adsorbent comprising the metal-organic framework is in a form of powder, granules, flakes, or pellets.
[13] The method according to any one of [1] to [12], wherein the first mixture is an azeotropic mixture or pseudoazeotropic mixture of fluorocarbons.
[14] The method according to any one of [1] to [13], further comprising: bringing the separated second mixture and the adsorbent comprising the metal-organic framework into contact with each other.
[15] The method according to any one of [1] to [14], further comprising: purifying the separated second mixture.
[16] The method according to any one of [1] to [15], further comprising, after bringing the first mixture and the adsorbent comprising the metal-organic framework into contact with each other:
   desorbing the fluorocarbon A adsorbed to the adsorbent comprising the metal-organic framework from the adsorbent comprising the metal-organic framework.
[17] A fluorocarbon purification system that performs the separation method according to any one of [1] to [16].
[18] A method for separating a fluorocarbon, comprising:
   separating, from a first mixture comprising a fluorocarbon A having n carbon atoms and one or more double bonds and a fluorocarbon B having m carbon atoms and no double bonds,
   a third mixture having a reduced concentration of the fluorocarbon A as compared with the first mixture,
   wherein the separation is performed by bringing the first mixture and an adsorbent into contact with each other.
[19] The method according to [18], wherein the first mixture and the adsorbent are brought into contact with each other at a temperature of -10°C or higher and 80°C or lower.
[20] The method according to [18] or [19], wherein n is 2 or more and 3 or less.
[21] The method according to any one of [18] to [20], wherein m is 1 or more and 3 or less.
[22] The method according to any one of [18] to [21], wherein the fluorocarbon A comprises R1234yf.
[23] The method according to any one of [18] to [22], wherein the fluorocarbon B comprises one or more selected from R134a, R143, R143a, R32 and R125.
[24] The method according to any one of [18] to [23], wherein the adsorbent comprises a porous body.
[25] The method according to any one of [18] to [24], wherein the adsorbent comprises a metal-organic framework.
[26] The method according to [25], wherein the metal-organic framework comprises a metal ion.
[27] The method according to [25], wherein the metal-organic framework comprises one or more organic ligands, and the organic ligand comprises two or more groups capable of forming a coordinate bond to a metal ion, per molecule.
[28] The method according to [25], wherein the metal-organic framework comprises a metal ion and one or more organic ligands, and the organic ligand comprises two or more groups capable of forming a coordinate bond to the metal ion, per molecule.
[29] The method according to [25], wherein the metal-organic framework comprises an open metal site provided by a coordinatively unsaturated metal ion.
[30] The method according to any one of [18] to [29], wherein the adsorbent further comprises a resin.
[31] The method according to any one of [18] to [30], wherein the adsorbent is in a form of powder, granules, flakes, or pellets.
[32] The method according to any one of [18] to [31], wherein the first mixture is an azeotropic mixture or pseudoazeotropic mixture of fluorocarbons.
[33] The method according to any one of [18] to [32], further comprising: bringing the separated third mixture and an adsorbent into contact with each other.
[34] The method according to any one of [18] to [33], further comprising: purifying the separated third mixture.
[35] The method according to any one of [18] to [34], further comprising, after bringing the first mixture and the adsorbent into contact with each other:
   desorbing the fluorocarbon A adsorbed to the adsorbent from the adsorbent.
[36] A fluorocarbon purification system that performs the separation method according to any one of [18] to [35].
[37] A composite material comprising: a metal-organic framework; and a fluorocarbon A having n carbon atoms and one or more double bonds,
   wherein the metal-organic framework comprises an open metal site provided by a coordinatively unsaturated metal ion.

### Advantageous Effects

According to the present disclosure, a novel method for separating a fluorocarbon can be provided.

### Brief Description of Drawings

[Figure 1] Figure 1 is a schematic view showing a fluorocarbon purification system in one embodiment of the present disclosure.
[Figure 2] Figure 2 is a schematic view showing a fluorocarbon purification system in another embodiment of the present disclosure.
[Figure 3] Figure 3 shows adsorption breakthrough curves in Example 1 and Comparative Example 1.

### Description of Embodiments

### (First embodiment: First separation method)

A first separation method of the present disclosure comprises:
separating, from a first mixture comprising a fluorocarbon A having n carbon atoms and one or more double bonds and a fluorocarbon B having m carbon atoms and no double bonds,
a second mixture in which the composition ratio between the fluorocarbon A and the fluorocarbon B is different from that in the first mixture, and
the separation is performed by bringing the first mixture and an adsorbent comprising a metal-organic framework into contact with each other.

According to the first separation method of the present disclosure, the adsorbent comprising a metal-organic framework and the first mixture comprising two or more kinds of fluorocarbon gases are brought into contact with each other, thereby separating the second mixture having a reduced proportion of one of the fluorocarbon gas having a double bond or a fluorocarbon gas having no double bonds. Without being bound to a particular theory, in the present invention, the pore structure of the metal-organic framework is considered to control the adsorption behavior between the fluorocarbon A having a double bond or the fluorocarbon B having no double bonds and the metal-organic framework, thereby providing a mixture having a different composition ratio. In some cases, either one of the fluorocarbon A or the fluorocarbon B may not be comprised in the second mixture.

In one embodiment, the first separation method comprises: separating, from a first mixture comprising a fluorocarbon A having n carbon atoms and one or more double bonds and a fluorocarbon B having m carbon atoms and no double bonds,
a second mixture having a reduced concentration of the fluorocarbon A as compared with the first mixture,
wherein the separation is performed by bringing the first mixture and an adsorbent comprising a metal-organic framework into contact with each other.

In another embodiment, the first separation method comprises separating, from a first mixture comprising a fluorocarbon A having n carbon atoms and one or more double bonds and a fluorocarbon B having m carbon atoms and no double bonds,
a second mixture having a reduced concentration of the fluorocarbon B as compared with the first mixture, and
the separation is performed by bringing the first mixture and an adsorbent comprising a metal-organic framework into contact with each other.

### (First mixture)

The first mixture comprises a fluorocarbon A having n carbon atoms and one or more double bonds and a fluorocarbon B having m carbon atoms and no double bonds. In the present embodiment, the fluorocarbon A or the fluorocarbon B is also referred to as an adsorbate.

In the fluorocarbon A, the number of the double bonds is 1 or more and can be preferably 1 or more and 5 or less, more preferably 1 or more and 3 or less, and still more preferably 1 or more and 2 or less.

The number of carbon atoms n in the fluorocarbon A is preferably 2 or more and 10 or less, more preferably 2 or more and 5 or less, and still more preferably 2 or more and 3 or less.

The boiling point of the fluorocarbon A may be, for example, -80°C or higher and -20°C or lower and, furthermore, may be -60°C or higher and -30°C or lower. In one embodiment, the fluorocarbon having n carbon atoms is present in the form of a gas in the first mixture.

Examples of the fluorocarbon A comprise hydrochlorofluoroolefin and hydrofluoroolefin.

Examples of the hydrofluoroolefin comprise 1,1-difluoroethylene, 2,3,3,3-tetrafluoropropene, 1,3,3,3-tetrafluoropropene, hexafluoropropene, and 1,1,1,4,4,4-hexafluorobutene. The fluorocarbon A is preferably hydrofluorocarbon and more preferably 2,3,3,3-tetrafluoropropene (R1234yf).

Examples of the hydrochlorofluoroolefin comprise 1-chloro-3,3,3-trifluoropropene and 1-chloro-2,3,3,3-tetrafluoropropene.

The fluorocarbon A may comprise one or more compounds.

The concentration of the fluorocarbon A in the first mixture may be, for example, 0.0001 mol% or higher and 90 mol% or lower or, furthermore, may be 0.0001 mol% or higher or 20 mol% or lower in the entire first mixture.

The number of carbon atoms m in the fluorocarbon B is preferably 1 or more and 10 or less, more preferably 1 or more and 5 or less, and still more preferably 1 or more and 3 or less.

The difference between the number of carbon atoms in the fluorocarbon A and the number of carbon atoms in the fluorocarbon B may be, for example, 0 or more and 5 or less, furthermore, may be 0 or more and 3 or less, and particularly, may be 0 or more and 1 or less. The separation method of the present disclosure can separate the second mixture having a different composition ratio even when the numbers of carbon atoms in the fluorocarbon A and the fluorocarbon B are close to each other.

The boiling point of the fluorocarbon B may be, for example, -80°C or higher and -20°C or lower and, furthermore, may be -60°C or higher and -30°C or lower. In one embodiment, the fluorocarbon having n carbon atoms is present in the form of a gas in the first mixture.

The difference between the boiling point of the fluorocarbon A and the boiling point of the fluorocarbon B may be, for example, 0°C or more and 20°C or less and, furthermore, may be 0°C or more and 10°C or less. The separation method of the present disclosure can separate the second mixture having a different composition ratio even when the boiling points of the fluorocarbon A and the fluorocarbon B are close to each other.

Examples of the fluorocarbon B comprise hydrochlorofluorocarbon and hydrofluorocarbon.

Examples of the hydrofluorocarbon comprise difluoromethane, 1,1-difluoroethane, 1,2-difluoroethane, 1,1,1-trifluoroethane, 1,1,2-trifluoroethane, 1,1,1,2-tetrafluoroethane, 1,1,2,2-tetrafluoroethane, and pentafluoroethane.

Examples of the hydrochlorofluorocarbon comprise chlorodifluoromethane and chlorotrifluoroethane.

The fluorocarbon B is preferably the hydrofluorocarbon and more preferably one or more selected from 1,1,1,2-tetrafluoroethane (R134a), 1,1,1-trifluoroethane (R143a), and 1,1,1,2,2-pentafluoroethane (R125).

The fluorocarbon B may comprise one or more compounds.

The concentration of the fluorocarbon B in the first mixture may be, for example, 0.0001 mol% or higher and 90 mol% or lower or, furthermore, may be 0.0001 mol% or higher or 20 mol% or lower in the entire first mixture.

When the composition ratio of the fluorocarbon A and the fluorocarbon B, (A/ (A + B) × 100) in the first composition is represented by C₁, C₁ may be, for example, 0.0001 or more and 90 or less, more preferably 0.0001 or more and 50 or less, and still more preferably 0.0001 or more and 20 or less on a molar basis.

The first mixture may be an azeotropic mixture or pseudoazeotropic mixture of fluorocarbons. According to the separation method of the present disclosure, a fluorocarbon having a specific number of carbon atoms can be separated even when the mixture is an azeotropic mixture or a pseudoazeotropic mixture. Examples of such an azeotropic mixture or pseudoazeotropic mixture comprise mixtures in which the difference between the boiling point of a fluorocarbon that is an separation target (in the present embodiment, the fluorocarbon A or the fluorocarbon B) and the boiling point of the other fluorocarbon(s) is 1°C or more and 20°C or less, furthermore, 3°C or more and 18°C or less at 1 atmosphere (1,013 hPa).

In one embodiment, the fluorocarbon A may be 2,3,3,3-tetrafluoropropene (R1234yf), and the fluorocarbon B may be one or more selected from 1,1,1,2-tetrafluoroethane (R134a), 1,1,1-trifluoroethane (R143a), and 1,1,1,2,2-pentafluoroethane (R125).

The first mixture may comprise a different compound, in addition to the fluorocarbon A and the fluorocarbon B. The boiling point of such a different compound may be, for example, -100°C or lower or may be -150°C or lower. In one embodiment, such a different compound is comprised as a gas in the first mixture.

Examples of the different compound comprise nitrogen, oxygen, carbon dioxide, and water.

### (Adsorbent)

The adsorbent comprises a metal-organic framework (MOF or porous coordination polymer (PCP)). In the present embodiment, the adsorbent comprising the metal-organic framework will also be simply referred to as the adsorbent.

The metal-organic framework can be typically an organic/inorganic composite framework having a regularly continuous three-dimensional skeleton. The metal-organic framework preferably comprises a metal ion and one or more organic ligands. Typically, the organic ligand preferably comprises two or more groups capable of forming a coordinate bond to the metal ion, per molecule. When the organic ligand coordinates to the metal ion, a highly regular and three-dimensionally continuous structure (for example, a network structure) is formed, and a pore is thus formed between the metal ion and the organic ligand. By combining the kind of the metal ion and the organic ligand, the effective pore diameter or flexibility of the metal-organic framework and the interaction between the metal-organic framework and the adsorbate can be controlled in such a three-dimensional structure.

In addition, the metal-organic framework has an adsorption capacity (maximum adsorption capacity) that does not decrease even after the repeated adsorption and desorption of the adsorbate and thus has high durability to repetition. Furthermore, the metal-organic framework is less likely to be affected by temperatures (heat) or humidity, sustaining an adsorption capacity that does not decrease even through storage at a high temperature and a high humidity for a long period of time and thus exhibits high durability to high-temperatures and high-humidities.

The pore structure of the metal-organic framework is considered to control the adsorption behavior between the fluorocarbon A having a double bond or the fluorocarbon B having no double bonds and the metal-organic framework thereby changing the composition ratio of the fluorocarbon A and the fluorocarbon B in the first mixture.

The metal ion is not limited, and examples thereof comprise the ions of metals selected from the group consisting of Group Ia, Group IIa, Group IIIa, Groups IVa to VIII and Groups Ib to VIb. Such metals can be preferably one or more selected from the group consisting of Mg, Ca, Sr, Ba, Sc, Y, Ti, Zr, Hf, V, Nb, Ta, Cr, Mo, W, Mn, Re, Fe, Ro, Os, Co, Rh, Ir, Ni, Pd, Pt, Cu, Ag, Au, Zn, Cd, Hg, Al, Ga, In, Tl, Si, Ge, Sn, Pb, As, Sb, Bi, La, Ce, Pr, Nd, Pm, Sm, En, Gd, Tb, Dy, Ho, Er, Tm and Yb and can be more preferably one or more selected from the group consisting of Zn, Cu, Ti, Co, Mn, Ni, Al, Ca, Zr and Mg.

Specifically, such metal ions can be one or more selected from the group consisting of Mg²⁺, Ca²⁺, Sr²⁺, Ba²⁺, Sc³⁺, Y³⁺, Ln³⁺, Ti⁴⁺, Zr⁴⁺, Hf⁴⁺, V⁴⁺, V³⁺, V²⁺, Nb³⁺, Ta³⁺, Cr³⁺, Mo³⁺, W³⁺, Mn³⁺, Mn²⁺, Re³⁺, Re²⁺, Fe³⁺, Fe²⁺, Ru³⁺, Ru²⁺, Os³⁺, Os²⁺, Co³⁺, Co²⁺, Rh²⁺, Rh⁺, Ir²⁺, Ir⁺, Ni²⁺, Ni⁺, Pd²⁺, Pd⁺, Pt²⁺, Pt⁺, Cu²⁺, Cu⁺, Ag⁺, Au⁺, Zn²⁺, Cd²⁺, Hg²⁺, Al³⁺, Ga³⁺, In³⁺, T1³⁺, Si⁴⁺, Si²⁺, Ge⁴⁺, Ge²⁺, Sn⁴⁺, Sn²⁺, Pb⁴⁺, Pb²⁺, As⁵⁺, As^{3+,} As⁺, Sb⁵⁺, Sb³⁺, Sb⁺, Bi⁵⁺, Bi³⁺, Bi⁺, La³⁺ Ce³⁺, Pr³⁺, Nd³⁺, Pm³⁺, Sm³⁺, En³⁺, Gd³⁺, Tb³⁺, Dy³⁺, Ho³⁺, Er³⁺, Tm³⁺ and Yb³⁺ and can be more preferably one or more selected from the group consisting of Cr³⁺, Zn²⁺, Cu²⁺, Cu⁺, Ti⁴⁺, Ti³⁺, Co³⁺, Co²⁺, Mn³⁺, Mn²⁺, Fe³⁺, Fe²⁺, Ni²⁺, Ni⁺, Al³⁺, Ca²⁺, Zr⁴⁺ and Mg²⁺.

The number of types of the metal ions may be only 1 or 2 or more. The number of types of the metal ions is preferably 1.

In a preferable embodiment, the metal ion is one or more selected from a Fe ion, a Cu ion, a Ni ion, a Mg ion, a Co ion, a Cr ion, a Zr ion, and a Zn ion, and preferably one or more selected from Fe²⁺, Fe³⁺, Cu²⁺, Ni²⁺, Mg²⁺, Co²⁺, Cr³⁺, and Zn²⁺.

The organic ligand is not limited, as long as the organic ligand comprises two or more groups capable of forming a coordinate bond to the metal ion, per molecule. The coordinate bond is formed of, for example, a functional group capable of forming a coordinate bond to the metal ion.

Examples of the functional group capable of forming the coordinate bond comprise -COOH, -CS₂H, -NO₂, -B(OH)₂, -SO₃H, -Si(OH)₃, -Ge(OH)₃, -Sn(OH)₃, -Si(SH)₄, -Ge(SH)₄, - Sn(SH)₃, -PO₃H, -AsO₃H, -AsO₄H, -P(SH)₃, -As(SH)₃, - CH(RSH)₂, -C(RSH)₃, -CH (RNH₂)₂, -C(RNH₂)₃, -CH(ROH)₂, - C(ROH)₃, -CH(RCN)₃ and -C(RCN)₃. In the formulae, R is a single bond, an alkylene group having 1 to 5 carbon atoms (for example, methylene, ethylene, n-propylene, i-propylene, n-butylene, i-butylene, tert-butylene or a n-pentylene group), a divalent aromatic group having 6 to 14 carbon atoms (for example, for example phenylene) or a combination of the alkylene group and the aromatic group (for example, -phenylene-alkylene-phenylene). Alternatively, the functional group capable of forming a coordinate bond may be a hetero atom included in a heterocycle (for example, N, O, S, B, P, Si or Al) and may be preferably a nitrogen atom included in a heterocycle.

In a preferable embodiment, the functional group capable of forming a coordinate bond can be -COOH, or a nitrogen atom included in a heterocycle, for example.

The organic ligand preferably comprise the functional group so as to be a bi- or higher-dentate ligand. In such an organic ligand, the moiety other than the functional group is not limited as long as the organic ligand is capable of forming a coordinate bond to the metal ion.

In one embodiment, the organic ligand is derived from a saturated or unsaturated aliphatic compound or aromatic compound. The ligand may be derived from a compound formed by bonding such an aliphatic compound to an aromatic compound bond (hereinafter, also referred to as "aliphatic aromatic compound").

An aliphatic moiety of the aliphatic compound or the aliphatic aromatic compound may be linear, branched or cyclic. In the case where the aliphatic moiety is cyclic, the aliphatic moiety may have a plurality of rings. The aliphatic moiety of the aliphatic compound or the aliphatic aromatic compound preferably has 1 to 15 carbon atoms and more preferably has 1 to 10 carbon atoms, for example, 1, 2, 3, 4, 5, 6, 7, 8, 9 or 10 carbon atoms. In a preferable embodiment, the aliphatic moiety is derived from methane, adamantane, acetylene, ethylene or butadiene.

An aromatic moiety of the aromatic compound or the aromatic aliphatic compound may have 1 or more rings, for example, 2, 3, 4 or 5 rings. These rings may or may not be condensed. The aromatic moiety of the aromatic compound or the aliphatic aromatic compound preferably has 1, 2 or 3 rings and more preferably 1 or 2 rings. Each ring of the compound may have at least one heteroatom, for example, N, O, S, B, P, Si or Al, preferably N, O or S, in the ring. The aromatic moiety of the aromatic compound or the aromatic aliphatic compound preferably has 1 or 2 rings having 6 carbon atoms. In a case where there are 2 aromatic moieties, such two rings may or may not be condensed. In a preferable embodiment, such an aromatic moiety is derived from benzene, naphthalene, biphenyl, bipyridyl or pyridyl.

In one embodiment, the organic ligand is derived from dicarboxylic acid, tricarboxylic acid or tetracarboxylic acid.

Examples of the dicarboxylic acid comprise oxalic acid, succinic acid, tartaric acid, maleic acid, 1,4-butanedicarboxylic acid, 1,4-butenedicarboxylic acid, 4-oxopyran-2,6-dicarboxylic acid, 1,6-hexanedicarboxylic acid, decanedicarboxylic acid, 1,8-heptadecanedicarboxylic acid, 1,9-heptadecanedicarboxylic acid, heptadecanedicarboxylic acid, acetylenedicarboxylic acid, 1,2-benzenedicarboxylic acid, 1,3-benzenedicarboxylic acid, 2,3-pyridinedicarboxylic acid, pyridine-2,3-dicarboxylic acid, 1,3-butadiene-1,4-dicarboxylic acid, 1,4-benzenedicarboxylic acid, p-benzenedicarboxylic acid, imidazole-2,4-dicarboxylic acid, 2-methylquinoline-3,4-dicarboxylic acid, quinoline-2,4-dicarboxylic acid, quinoxaline-2,3-dicarboxylic acid, 6-chloroquinoxaline-2,3-dicarboxylic acid, 4,4'-diaminophenylmethane-3,3'-dicarboxylic acid, quinoline-3,4-dicarboxylic acid, 7-chloro-4-hydroxyquinoline-2,8-dicarboxylic acid, diimidodicarboxylic acid, pyridine-2,6-dicarboxylic acid, 2-methylimidazole-4,5-dicarboxylic acid, thiophene-3,4-dicarboxylic acid, 2-isopropylimidazole-4,5-dicarboxylic acid, tetrahydropyran-4,4-dicarboxylic acid, perylene-3,9-dicarboxylic acid, perylene dicarboxylic acid, PLURIOL E200-dicarboxylic acid, 3,6-dioxaoctanedicarboxylic acid, 3,5-cyclohexadiene-1,2-dicarboxylic acid, octanedicarboxylic acid, pentane-3,3-dicarboxylic acid, 4,4'-diamino-1,1'-biphenyl-3,3'-dicarboxylic acid, 4,4'-diaminobiphenyl-3,3'-dicarboxylic acid, benzidine-3,3'-dicarboxylic acid, 1,4-bis(phenylamino)benzene-2,5-dicarboxylic acid, 1,1'-binaphthyldicarboxylic acid, 7-chloro-8-methylquinoline-2,3-dicarboxylic acid, 1-anilino-anthraquinone-2,4'-dicarboxylic acid, polytetrahydrofuran 250-dicarboxylic acid, 1,4-bis(carboxymethyl)piperazine-2,3-dicarboxylic acid, 7-chloroquinoline-3,8-dicarboxylic acid, 1-(4-carboxy)phenyl-3-(4-chloro)phenylpyrazoline-4,5-dicarboxylic acid, 1,4,5,6,7,7-hexachloro-5-norbornene-2,3-dicarboxylic acid, phenylindanedicarboxylic acid, 1,3-dibenzyl-2-oxoimidazolidine-4,5-dicarboxylic acid, 1,4-cyclohexanedicarboxylic acid, naphthalene-1,8-dicarboxylic acid, 2-benzoylbenzene-1,3-dicarboxylic acid, 1,3-dibenzyl-2-oxoimidazolidene-4,5-cis-dicarboxylic acid, 2,2'-biquinoline-4,4'-dicarboxylic acid, pyridine-3,4-dicarboxylic acid, 3,6,9-trioxaundecanedicarboxylic acid, hydroxybenzophenone dicarboxylic acid, PLURIOL E300-dicarboxylic acid, PLURIOL E400-dicarboxylic acid, PLURIOL E600-dicarboxylic acid, pyrazole-3,4-dicarboxylic acid, 2,3-pyrazinedicarboxylic acid, 5,6-dimethyl-2,3-pyrazinedicarboxylic acid, 4,4'-diamino(diphenyl ether)diimidodicarboxylic acid, 4,4'-diaminodiphenylmethanediimidodicarboxylic acid, 4,4'-diamino(diphenylsulfone)diimidodicarboxylic acid, 1,4-naphthalene dicarboxylic acid, 2,6-naphthalene dicarboxylic acid, 1,3-adamantanedicarboxylic acid, 1,8-naphthalene dicarboxylic acid, 2,3-naphthalene dicarboxylic acid, 8-methoxy-2,3-naphthalene dicarboxylic acid, 8-nitro-2,3-naphthalene dicarboxylic acid, 8-sulfo-2,3-naphthalene dicarboxylic acid, anthracene-2,3-dicarboxylic acid, 2',3'-diphenyl-p-terphenyl-4,4''-dicarboxylic acid, (diphenyl ether)-4,4'-dicarboxylic acid, imidazole-4,5-dicarboxylic acid, 4(1H)-oxothiochromene-2,8-dicarboxylic acid, 5-tert-butyl-1,3-benzenedicarboxylic acid, 7,8-quinolinedicarboxylic acid, 4,5-imidazoledicarboxylic acid, 4-cyclohexene-1,2-dicarboxylic acid, hexatriacontanedicarboxylic acid, tetradedocanedicarboxylic acid, 1,7-heptanedicarboxylic acid, 5-hydroxy-1,3-benzenedicarboxylic acid, 2,5-dihydroxy-1,4-benzenedicarboxylic acid, pyrazine-2,3-dicarboxylic acid, furan-2,5-dicarboxylic acid, 1-nonene-6,9-dicarboxylic acid, eicosene dicarboxylic acid, 4,4'-dihydroxy-3,3'-biphenyldicarboxylic acid, 4,4'-dihydroxydiphenylmethane-3,3'-dicarboxylic acid, 1-amino-4-methyl-9,10-dioxo-9,10-dihydroanthracene-2,3-dicarboxylic acid, 2,5-pyridinedicarboxylic acid, cyclohexene-2,3-dicarboxylic acid, 2,9-dichlorofluorobin-4,11-dicarboxylic acid, 7-chloro-3-methylquinoline-6,8-dicarboxylic acid, 2,4-dichlorobenzophenone-2',5'-dicarboxylic acid, 1,3-benzenedicarboxylic acid, 2,6-pyridinedicarboxylic acid, 1-methylpyrrole-3,4-dicarboxylic acid, 1-benzyl-1H-pyrrole-3,4-dicarboxylic acid, anthraquinone-1,5-dicarboxylic acid, 3,5-pyrazoledicarboxylic acid, 2-nitrobenzene-1,4-dicarboxylic acid, heptane-1,7-dicarboxylic acid, cyclobutane-1,1-dicarboxylic acid, 1,14-tetradedocanedicarboxylic acid, 5,6-dehydronorbornane-2,3-dicarboxylic acid, 5-ethyl-2,3-pyridinedicarboxylic acid and camphordicarboxylic acid.

Examples of the tricarboxylic acid comprise 2-hydroxy-1,2,3-propanetricarboxylic acid, 7-chloro-2,3,8-quinolinetricarboxylic acid, 1,2,3-, 1,2,4-benzenetricarboxylic acid, 1,2,4-butanetricarboxylic acid, 2-phosphono-1,2,4-butanetricarboxylic acid, 1,3,5-benzenetricarboxylic acid, 4,4',4"-(1,3,5-benzenetriyl)trisbenzoic acid, 1-hydroxy-1,2,3-propanetricarboxylic acid, 4,5-dihydro-4,5-dioxo-1H-pyrrolo[2,3-F]quinoline-2,7,9-tricarboxylic acid, 5-acetyl-3-amino-6-methylbenzene-1,2,4-tricarboxylic acid, 3-amino-5-benzoyl-6-methylbenzene-1,2,4-tricarboxylic acid, 1,2,3-propane tricarboxylic acid and aurintricarboxylic acid.

Examples of the tetracarboxylic acid comprise perylenetetracarboxylic acids such as 1,1-dioxideperylo[1,12-BCD]thiophene-3,4,9,10-tetracarboxylic acid, perylene-3,4,9,10-tetracarboxylic acid or (perylene-1,12-sulfone)-3,4,9,10-tetracarboxylic acid, butanetetracarboxylic acids such as 1,2,3,4-butanetetracarboxylic acid or meso-1,2,3,4-butanetetracarboxylic acid, decane-2,4,6,8-tetracarboxylic acid, 1,4,7,10,13,16-hexaoxacyclooctadecane-2,3,11,13-tetracarboxylic acid, 1,2,4,5-benzenetetracarboxylic acid, 1,2,11,12-dodecanetetracarboxylic acid, 1,2,5,6-hexanetetracarboxylic acid, 1,2,7,8-octanetetracarboxylic acid, 1,4,5,8-naphthalenetetracarboxylic acid, 1,2,9,10-decanetetracarboxylic acid, benzophenonetetracarboxylic acid, 3,3',4,4'-benzophenonetetracarboxylic acid, tetrahydrofurantetracarboxylic acid and cyclopentanetetracarboxylic acids such as cyclopentane-1,2,3,4-tetracarboxylic acid.

In one embodiment, the organic ligand is derived from a heterocycle capable of forming a coordinate bond at the ring heteroatom. Examples of such a heterocycle comprise the following heterocycles. The heterocycle may be unsubstituted or substituted.

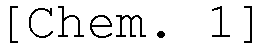

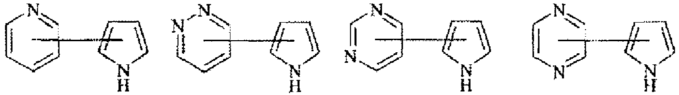

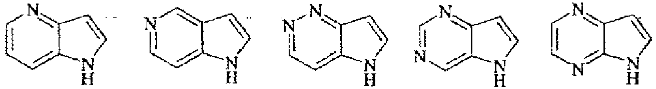

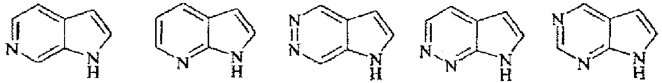

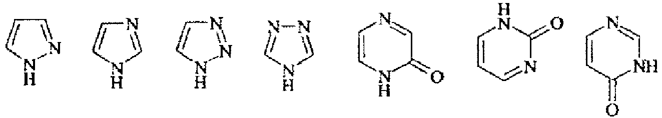

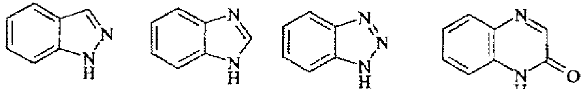

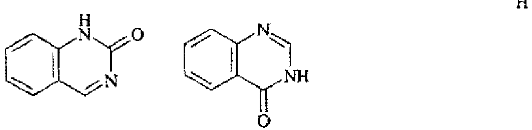

In one embodiment, the organic ligand is derived from an azole such as a diazole or a triazole and preferably derived from a triazole.

In a preferable embodiment, the organic ligand comprises an organic ligand derived from a polycarboxylic acid and an organic ligand derived from an azole and preferably comprises an organic ligand derived from an aromatic polycarboxylic acid.

In a preferable embodiment, the organic ligand is selected from 1,4-benzenedicarboxylic acid, 1,2-benzenedicarboxylic acid, maleic acid, 1,3,5-benzenetricarboxylic acid, 4,4',4"-(1,3,5-benzenetriyl)trisbenzoic acid, 2,5-dihydroxy-1,4-benzenedicarboxylic acid, oxalic acid, 4,4'-dihydroxy-3,3'-biphenyldicarboxylic acid, 2-hydroxy-1,2,3-propanetricarboxylic acid, 4,4'-bipyridine, triazole, imidazole, 3,3'-bipyrazole, benzimidazole, and 3,5-pyridinedicarboxylic acid. In a more preferable embodiment, the organic ligand is selected from 1,4-benzenedicarboxylic acid, 4,4'-dihydroxy-3,3'-biphenyldicarboxylic acid, 2-hydroxy-1,2,3-propanetricarboxylic acid, 1,3,5-benzenetricarboxylic acid, and 2,5-dihydroxy-1,4-benzenedicarboxylic acid.

The number of types of the organic ligands may be only one, or two or more.

In the present disclosure, "being derived from a compound" may encompass not only the compound itself but also a partially protonated form of such a compound and a fully protonated form of such a compound.

The metal-organic framework preferably comprise an open metal site provided by a coordinatively unsaturated metal ion. The open metal site means a site in which the metal comprised in the metal-organic framework is in a coordinatively unsaturated state and is thus capable of coordinating an arbitrary molecule. The presence or absence of the open metal site does not rely on the kinds of the metal ion and the organic ligand.

Examples of the metal-organic framework comprising the open metal site comprise HKUST-1, MIL-100 (Fe), MIL-101 (Cr), MOF-74 (Ni), MOF-74 (Mg), MOF-74 (Co), MOF-74 (Cu), Mg₂ (dobpdc), and UTSA-16 (Zn).

The metal-organic framework can be produced by bringing the metal ion and the organic ligand into contact with each other.

Such contact between the metal ion and the organic ligand may be performed in the presence of a solvent. The solvent can be water, ethanol, dimethylformamide, toluene, methanol, chlorobenzene, diethylformamide, dimethylsulfoxide, water, hydrogen peroxide, methylamine, a sodium hydroxide solution, N-methylpyrrolidone ether, acetonitrile, benzyl chloride, triethylamine, ethylene glycol or a mixture thereof.

The contact between the metal ion and the organic ligand may be performed under increased pressure or under normal pressure. The temperature at the time of performing such contact may be, for example, 10°C to 200°C or 10°C to 150°C. During such contact, stirring may be performed.

The effective pore diameter is, for example, 0.30 nm or more and 5.00 nm or less, preferably 0.4 nm or more and 4.00 nm or less, and more preferably 1.00 nm or more and 2.50 nm or less. In one embodiment, the effective pore diameter of the porous body is, for example, 0.30 nm or more, preferably 0.4 nm or more, and more preferably 1.00 nm or more and is, for example, 5.00 nm or less, preferably 4.00 nm or less, and more preferably 2.00 nm or less.

The effective pore diameter is the pore diameter of the porous body that is determined from the degree of adsorption and the molecular diameter of the adsorbate when the porous body such as the metal-organic framework and the adsorbate are brought into contact with each other and, specifically, can be obtained by converting the relative pressure of a nitrogen adsorption isotherm into the effective pore diameter using a Horvath-Kawazoe method. As a conversion formula, a calculation formula described in J. Chem. Eng, Jpn., 1983, 16, 6, 470 to 475 may be used, and as a parameter value in the conversion formula, a value based on a combination of carbon and nitrogen may be used.

The specific surface area of the metal-organic framework can be, for example, 40 m²/g or more, preferably 100 m²/g or more, and more preferably 300 m²/g or more and may be, for example, 3,000 m²/g or less and may be 2,000 m²/g or less. The specific surface area of the metal-organic framework is calculated from the nitrogen gas adsorption isotherm using a BET method.

The amount of the fluorocarbon A or the fluorocarbon B adsorbed in equilibrium to the metal-organic framework may be, for example, 1 g/10 g or more or 100 g/10 g or less as measured at a temperature of 298 K and a pressure within a range of 0.4 PaG to 1 MPaG (gauge pressure).

The content of the metal-organic framework in the adsorbent is, for example, 50 mass% or more and 100 mass% or less, preferably 70 mass% or more and 100 mass% or less and more preferably 90 mass% or more and 100 mass% or less.

The absorbent may comprise a resin in addition to the metal-organic framework. Examples of such a resin comprise an acrylic resin, a polyurethane resin, a polyolefin resin, a polyester resin, a polyamide resin, a vinyl chloride resin, a styrene resin, a vinyl ether resin, a polyvinyl alcohol resin, a polycarbonate resin, and a polysulfone resin.

The absorbent may comprise an additive such as an emulsifier, an antifoaming agent, a surfactant, a leveling agent, a thickener, a viscoelastic modifier, an antifoaming agent, a wetting agent, a dispersant, a preservative, a plasticizer, a penetrant, a fragrance, a bactericide, an acaricide, a fungicide, an ultraviolet absorber, an antioxidant, an antistatic agent, a flame retarder, a dye or a pigment. The adsorbent may be in the form of powder, granules, flakes, or pellets.

### (Contact between first mixture and adsorbent)

When the first mixture and the adsorbent are brought into contact with each other, the fluorocarbon A or the fluorocarbon B can be adsorbed to the metal-organic framework that is comprised in the adsorbent, and it is thus possible to separate the second mixture in which the composition ratio of the fluorocarbon A and the fluorocarbon B is different from that in the first mixture from the first mixture. In one embodiment, the first mixture is preferably in the form of a gas when the first mixture and the adsorbent are brought into contact with each other.

The contact between the first mixture and the adsorbent may be performed by, for example, a batch method or a column method. The batch method is a method in which the adsorbent and the first mixture are put into a sealable container and left still for a certain period of time under conditions of a predetermined temperature and a predetermined pressure, thereby bringing the first mixture and the adsorbent into contact with each other. The column method is a method in which a container (column) is packed with the adsorbent and the first mixture is made to flow into the container (column), thereby bringing the first mixture and the adsorbent into contact with each other.

In one embodiment, the temperature at the time of the contact may be, for example, 0°C or higher and 100°C or lower and, furthermore, 0°C or higher and 50°C or lower. In another embodiment, the temperature at the time of the contact may be, for example, 10°C or higher and 50°C or lower, 15°C or higher and 50°C or lower or 15°C or higher and 30°C or lower. The pressure (gauge pressure) at the time of the contact may be 0 MPaG or higher and 2 MPaG or lower or 0.2 MPaG or higher and 1.5 MPaG or lower. When the pressure at the time of the contact is within the above-described range, it is easy for the adsorbate to be adsorbed to the adsorptive medium, so that the separation efficiency can improve, and the pore structure of the adsorptive medium can also be maintained.

When performing the contact by the column method, the linear velocity of the first mixture may be 0.01 cm/second or faster and 100 cm/second or slower or may be 0.05 cm/second or faster and 10 cm/second or slower.

### (Second mixture)

The second mixture comprises one or more selected from the fluorocarbon A and the fluorocarbon B, and the composition ratio of the fluorocarbon A and the fluorocarbon B is different from that in the first mixture. The second mixture preferably comprises the fluorocarbon A and the fluorocarbon B.

When the composition ratio of the fluorocarbon A and the fluorocarbon B, (A/ (A + B) × 100), in the second mixture is represented by C₂, C₂ may be, for example, 0.0001 or more and 10 or less, more preferably 0.0001 or more and 5 or less, and still more preferably 0.0001 or more and 1 or less on a molar basis.

The molar concentration of the fluorocarbon A in the second mixture, C_{A2}, may be, for example, 0.0001 mol% or higher and 1 mol% or lower, and furthermore, may be 0.0001 mol% or higher and 0.01 mol% or lower in the entire second mixture.

### (Regeneration of adsorbent)

The separation method of the present disclosure may further comprise desorbing the fluorocarbon A or the fluorocarbon B adsorbed to the adsorbent from the adsorbent after the first mixture and the adsorbent are brought into contact with each other. When the adsorbed fluorocarbon A or fluorocarbon B is desorbed from the adsorbent, the adsorption capability can be restored, and the adsorbent can be regenerated. This also makes it possible to collect the fluorocarbon A or the fluorocarbon B.

In one embodiment, the fluorocarbon that is desorbed from the adsorbent can be the fluorocarbon A. In another embodiment, the fluorocarbon that is desorbed from the adsorbent can be the fluorocarbon B.

The fluorocarbon A or the fluorocarbon B can be desorbed by, for example, depressurization, heating, the flow of air and nitrogen, or the flow of heated air and heated nitrogen.

The pressure (gauge pressure) at the time of the desorption can be preferably 0.1 MPaG or lower, more preferably 0 MPaG or lower, and still more preferably - 0.10 MPaG or lower. The temperature at the time of the desorption can be preferably 15°C to 200°C, more preferably 50°C to 180°C, and still more preferably 100°C to 150°C.

### (Contact between second mixture and adsorbent)

The separation method of the present disclosure may further comprise bringing the separated second mixture and the adsorbent into contact with each other. Such an operation makes it possible to obtain a new mixture in which the composition ratio of the fluorocarbon A and the fluorocarbon B is different from that in the second mixture. In one embodiment, when the second mixture has a reduced concentration of the fluorocarbon A as compared with the first mixture, the new mixture preferably has a reduced concentration of the fluorocarbon A as compared with the second mixture. In another embodiment, when the second mixture has a reduced concentration of the fluorocarbon B as compared with the first mixture, the new mixture preferably has a reduced concentration of the fluorocarbon B as compared with the second mixture.

The number of times of the contact between the adsorbent and the mixture is not limited, but can be preferably 1 to 5 and more preferably 1 to 3. In the present disclosure, the first contact corresponds to the contact between the first mixture and the adsorbent, and the m^{th} contact with the adsorbent corresponds to the contact between a mixture obtained by the (m-1)^{th} contact with the adsorbent and the adsorbent (wherein m is an integer of 2 or more). In the present embodiment, the adsorbents that are used in the second and subsequent contacts are adsorbents comprising a metal-organic framework, but the adsorbents are not limited thereto, and may be adsorbents comprising a porous body, which will be described for a second embodiment. The adsorbents that are used in two or more contacts may be the same as or different from one another.

The operation and conditions for bringing the mixture obtained by the (m-1)^{th} contact and the adsorbent into contact with each other can be performed in the same manner as the operation and conditions for bringing the first mixture and the adsorbent into contact with each other.

### (Purification)

The separation method of the present disclosure may further comprise purifying the separated second mixture. Such purification may comprise obtaining a new mixture in which the composition ratio of the fluorocarbon A and the fluorocarbon B is different from that in the second mixture. In one embodiment, when the second mixture has a reduced concentration of the fluorocarbon A compared to the first mixture, the new mixture preferably has a reduced concentration of the fluorocarbon A compared to the second mixture. In another embodiment, when the second mixture has a reduced concentration of the fluorocarbon B compared to the first mixture, the new mixture preferably has a reduced concentration of the fluorocarbon B compared to the second mixture.

The number of times of the purification is not limited, but can be preferably 1 to 5 and more preferably 1 to 3. In the present disclosure, the n^{th} purification means an operation for purifying the (n-1)^{th} new mixture obtained by the (n-1)^{th} purification (wherein n is an integer of 2 or more).

In one embodiment, the purification can be performed by distillation or rectification. Such distillation may be performed using a generally used distillation tower, for example, a packed tower or a tray tower. A purified product is distilled as a distillate from the top portion of the distillation tower, and the residue is distilled as a bottom from the bottom portion of the distillation tower. The number of theoretical plates of the distillation tower may be, for example, 1 to 100 plates. The pressure (gauge pressure) at the time of the distillation or rectification may be, 0 MPaG or higher and 5 MPaG or lower. The temperature of the top portion of the distillation tower may be, for example, -60°C or higher and 100°C or lower, and the temperature of the bottom portion of the distillation tower may be, for example, 50°C or higher and 200°C or lower. During the distillation, an extraction solvent may coexist.

The rectification can be performed by performing an operation of distilling the distillate again (operation of performing distillation twice or more).

In the present embodiment, the second mixture is purified, but the present disclosure is not limited thereto. For example, the first mixture may be purified before the first mixture and the adsorbent comprising the metal-organic framework are brought into contact with each other. That is, a mixture purified by the above operation may be brought into contact with the adsorbent comprising the metal-organic framework.

### (System)

A fluorocarbon purification system that performs the first separation method (hereinafter, also simply referred to as "system") is also comprised in the technical scope of the present disclosure.

The system preferably comprises
an adsorbent comprising a metal-organic framework, and
the adsorbent comprising the metal-organic framework is capable of separating, from a first mixture comprising a fluorocarbon A having n carbon atoms and one or more double bonds and a fluorocarbon B having m carbon atoms and no double bonds,
a second mixture in which the composition ratio between the fluorocarbon A and the fluorocarbon B is different from that in the first mixture.

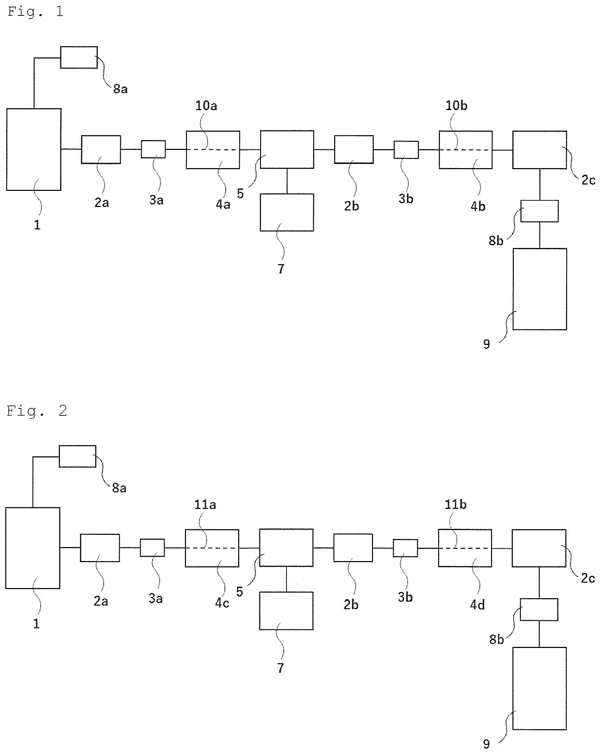

As one example of the system in the present embodiment, a two-stage type system is schematically shown in Figure 1, which will now be described.

In the system of Figure 1, the first mixture is stored in a gas tank 1. The first mixture is supplied to a first-stage separation module 4a through a pressure regulator 2a and a mass flow controller 3a. In one embodiment, the back pressure of the first mixture that is supplied to the separation module 4a can be controlled under increased pressure with the pressure regulator 2a, but is not limited to such an embodiment. The pressure regulator 2a may not be provided.

The separation module 4a comprises an adsorbent 10a comprising a metal-organic framework, and the first mixture is supplied to such an adsorbent 10a comprising a metal-organic framework.

In a two-stage type separation instrument, after the contact with the adsorbent 10a comprising a metal-organic framework, the flow rate of the second mixture is measured with the mass flow meter 5, and the second mixture is supplied to a second-stage separation module 4b through a pressure regulator 2b and a mass flow controller 3b. In one embodiment, the back pressure of the second mixture that is supplied to the second-stage separation module 4b can be controlled under increased pressure with the pressure regulator 2b, but is not limited to such an embodiment. The pressure regulator 2b may not be provided.

The second-stage separation module 4b comprises an adsorbent 10b comprising a metal-organic framework, and the second mixture is supplied to such an adsorbent 10b comprising a metal-organic framework.

A new mixture separated by the contact between the second mixture and the adsorbent 10b comprising a metal-organic framework is passed through a pressure regulator 2c, the gas composition is analyzed with a gas composition analyzer 8b, and then the new mixture is collected to a collection pipe 9. In one embodiment, the flow rate of the new mixture separated by the contact with the adsorbent 10b comprising a metal-organic framework may be measured using a mass flow meter. In one embodiment, a gas composition analyzer 8a may not be provided.

Although the two-stage separation instrument has been described in the present embodiment, the instrument is not limited thereto, and can be appropriately modified, and for example, the instrument may be a one-stage, three-stage or four or more-stage separation instrument.

In the case of a p-stage type (p is 3 or more), in a (p-1)^{th}-stage separation module, the (p-1)^{th} mixture separated by the contact with an adsorbent may be supplied to an separation module through a pressure regulator and a mass flow controller and may be brought into contact with an adsorbent that is comprised in the separation module to separate further a p^{th} new mixture. p^{th}-stage separation can be performed by collecting the p^{th} mixture after gas composition analysis that can be performed as necessary.

### (Composite material)

A composite material comprising a metal-organic framework and the fluorocarbon A or the fluorocarbon B is also included in the technical scope of the present disclosure. In such a composite material, the metal-organic framework preferably comprises an open metal site. In one embodiment, the composite material preferably comprises a metal-organic framework and the fluorocarbon **A.** In another embodiment, the composite material preferably comprises a metal-organic framework and the fluorocarbon **B.**

The content of the fluorocarbon A or the fluorocarbon B in the composite material is, for example, 0.1 parts by mass or more and 100 parts by mass or less and preferably 1 part by mass or more and 50 parts by mass or less, per 100 parts by mass of the metal-organic framework.

The composite material may comprise a resin, in addition to the fluorocarbon and a porous organic salt. Examples of such a resin comprise an acrylic resin, a polyurethane resin, a polyolefin resin, a polyester resin, a polyamide resin, a vinyl chloride resin, a styrene resin, a vinyl ether resin, a polyvinyl alcohol resin, a polycarbonate resin, and a polysulfone resin.

The composite material may comprise an additive such as an emulsifier, an antifoaming agent, a surfactant, a leveling agent, a thickener, a viscoelastic modifier, an antifoaming agent, a wetting agent, a dispersant, a preservative, a plasticizer, a penetrant, a fragrance, a bactericide, an acaricide, a fungicide, an ultraviolet absorber, an antioxidant, an antistatic agent, a flame retarder, a dye or a pigment.

### (Second embodiment: Second separation method)

A second separation method of the present disclosure comprises separating,
from a first mixture comprising a fluorocarbon A having n carbon atoms and one or more double bonds and a fluorocarbon B having m carbon atoms and no double bonds,
a third mixture comprising a reduced concentration of the fluorocarbon A as compared with the first mixture,
wherein the separation is performed by bringing the first mixture and an adsorbent into contact with each other.

According to the first separation method of the present disclosure, the adsorbent and the first mixture comprising two or more kinds of fluorocarbon gases are brought into contact with each other, thereby separating the third mixture having a reduced proportion of the fluorocarbon gas having a double bond. Without being bound to a particular theory, in the present invention, the adsorbent is considered to adsorb the fluorocarbon A having a double bond to obtain the third mixture comprising a reduced amount of the fluorocarbon A having a double bond. In some cases, the fluorocarbon A may not be comprised in the third mixture.

The first mixture, the fluorocarbon A, and the fluorocarbon B are the same meanings as the first mixture, the fluorocarbon A, and the fluorocarbon B in the first embodiment.

The carbon atom number n of the fluorocarbon A is preferably 2 or more and 10 or less, more preferably 2 or more and 5 or less, and still more preferably 2 or more and 3 or less.

The fluorocarbon A is preferably hydrofluoroolefin and more preferably 2,3,3,3-tetrafluoropropene (R1234yf).

The fluorocarbon A may comprise one or more compounds.

The concentration of the fluorocarbon A in the first mixture, C_{A1}, may be, for example, 0.0001 mol% or higher and 90 mol% or lower, and furthermore, may be 0.0001 mol% or higher and 20 mol% or lower in the entire first mixture.

The fluorocarbon B is preferably hydrofluorocarbon and more preferably one or more selected from 1,1,1,2-tetrafluoroethane (R134a), 1,1,1-trifluoroethane (R143a), and 1,1,1,2,2-pentafluoroethane (R125).

The fluorocarbon B may comprise one or more compounds.

The concentration of the fluorocarbon B in the first mixture may be, for example, 0.0001 mol% or higher and 90 mol% or lower or, furthermore, may be 0.0001 mol% or higher or 20 mol% or lower in the entire first mixture.

The first mixture may comprise a different compound, in addition to the fluorocarbon A and the fluorocarbon B. The boiling point of such a different compound may be, for example, -100°C or lower or may be -150°C or lower. In one embodiment, such a different compound is comprised as a gas in the first mixture.

Examples of the different compound comprise nitrogen, oxygen, carbon dioxide, and hydrogen.

### (Adsorbent)

The adsorbent (hereinafter, in the present embodiment, also referred to as "second adsorbent") is capable of adsorbing the fluorocarbon A having n carbon atoms and a double bond. Typically, the second adsorbent adsorbs the fluorocarbon A having a double bond, but does not adsorb the fluorocarbon B having no double bonds. Therefore, when the first mixture and the second adsorbent are brought into contact with each other, the third mixture comprising a reduced amount of fluorocarbon A can be separated from the first mixture.

The second adsorbent preferably comprises a porous body having a pore, as an adsorptive medium. The fluorocarbon having n carbon atoms can be adsorbed onto such a pore to thereby separate the fluorocarbon having n carbon atoms from the first mixture.

The effective pore diameter of the porous body is, for example, 0.30 nm or more and 5.00 nm or less, preferably 0.4 nm or more and 4.00 nm or less, and more preferably 1.00 nm or more and 2.50 nm or less. In one embodiment, the effective pore diameter of the porous body is, for example, 0.30 nm or more, preferably 0.40 nm or more, and more preferably 1.00 nm or more and is, for example, 5.00 nm or less, preferably 4.00 nm or less, and more preferably 2.00 nm or less.

The specific surface area of the porous body can be, for example, 40 m²/g or more, preferably 100 m²/g or more, and more preferably 300 m²/g or more and may be, for example, 3,000 m²/g or less and may be 2,000 m²/g or less. The specific surface area of the porous body is calculated from the nitrogen gas adsorption isotherm using a BET method.

The amount of the fluorocarbon having n carbon atoms adsorbed in equilibrium to the porous body may be, for example, 1 g/10 g or more or 100 g/10 g or less as measured at a temperature of 298 K and a pressure within a range of 0.4 PaG to 1 MPaG (gauge pressure).

The porous body may be in the form of powder (particles), a film, granules, or a molded product (for example, pellets), for example, and is preferably a molded product and more preferably in the form of pellets. A pellet may have a cylindrical shape or a spherical shape. In the case of the cylindrical shape, the radius of the cylinder is preferably 1.6 mm or more and 6 mm or less, and the thickness of the cylinder is preferably 2 mm or more and 6 mm or less. In the case of the spherical shape, the spherical shape does not need to be truly spherical at all times, and the radius of the sphere is preferably 4 mm or more and 12 mm or less.

The porous body preferably comprises, for example, at least one selected from the group consisting of zeolite, activated carbon, silica gel and a metal-organic framework (MOF, or PCP: porous coordination polymer), and typically comprises a metal-organic framework.

The metal-organic framework is the same meaning as the metal-organic framework in the first embodiment.

The content of the porous body in the second adsorbent is, for example, 50 mass% or more and 100 mass% or less, preferably 70 mass% or more and 100 mass% or less, and more preferably 90 mass% or more and 100 mass% or less.

The second adsorbent may comprise a resin in addition to the porous body. Examples of such a resin comprise an acrylic resin, a polyurethane resin, a polyolefin resin, a polyester resin, a polyamide resin, a vinyl chloride resin, a styrene resin, a vinyl ether resin, a polyvinyl alcohol resin, a polycarbonate resin, and a polysulfone resin.

The second adsorbent may comprise an additive such as an emulsifier, an antifoaming agent, a surfactant, a leveling agent, a thickener, a viscoelastic modifier, an antifoaming agent, a wetting agent, a dispersant, a preservative, a plasticizer, a penetrant, a fragrance, a bactericide, an acaricide, a fungicide, an ultraviolet absorber, an antioxidant, an antistatic agent, a flame retarder, a dye or a pigment. The second adsorbent may be in the form of powder, granules, flakes, or pellets.

### (Contact between first mixture and second adsorbent)

When the first mixture and the second adsorbent are brought into contact with each other, the fluorocarbon A that may be comprised in the first mixture to be adsorbed to the adsorbent to separate the third mixture having a reduced concentration of the fluorocarbon A from the first mixture. In one embodiment, the first mixture is preferably in the form of a gas when the first mixture and the second adsorbent are brought into contact with each other.

The contact between the first mixture and the second adsorbent may be performed by, for example, a batch method or a column method. The batch method is a method in which the second adsorbent and the first mixture are put into a sealable container and left still for a certain period of time under conditions of a predetermined temperature and a predetermined pressure, thereby bringing the first mixture and the second adsorbent into contact with each other. The column method is a method in which a container (column) is packed with the second adsorbent and the first mixture is made to flow into the container (column), thereby bringing the first mixture and the second adsorbent into contact with each other.

In one embodiment, the temperature at the time of the contact may be, for example, 0°C or higher and 100°C or lower and, furthermore, 0°C or higher and 50°C or lower. In another embodiment, the temperature at the time of the contact may be, for example, 10°C or higher and 50°C or lower, 15°C or higher and 50°C or lower or 15°C or higher and 30°C or lower. The pressure (gauge pressure) at the time of the contact may be 0 MPaG or higher and 2 MPaG or lower or 0.2 MPaG or higher and 1.5 MPaG or lower. When the pressure at the time of the contact is within the above-described range, it is easy for the adsorbate to be adsorbed to the adsorptive medium, so that the separation efficiency can improve, and the pore structure of the adsorptive medium can also be maintained.

In the case of performing the contact by the column method, the linear velocity of the first mixture may be 0.01 cm/second or faster and 100 cm/second or slower or may be 0.05 cm/second or faster and 10 cm/second or slower.

### (Third mixture)

The third mixture comprises at least the fluorocarbon B, and has a reduced concentration of the fluorocarbon A compared to the first mixture. The third mixture preferably comprises the fluorocarbon A and the fluorocarbon B.

When the composition ratio of the fluorocarbon A and the fluorocarbon B, (A/(A + B) × 100), in the third mixture is represented by C₃, C₃ may be, for example, 0.0001 or more and 10 or less, more preferably 0.0001 or more and 5 or less, and still more preferably 0.0001 or more and 1 or less on a molar basis.

The molar concentration of the fluorocarbon A in the third mixture, C_{A3}, may be, for example, 0.0001 mol% or higher and 1 mol% or lower, and furthermore, may be 0.0001 mol% or higher and 0.01 mol% or lower in the entire third mixture.

### (Regeneration of adsorbent)

The separation method of the present disclosure may further comprise desorbing the fluorocarbon A adsorbed to the second adsorbent from the second adsorbent after the first mixture and the second adsorbent are brought into contact with each other. When the adsorbed fluorocarbon A is desorbed from the second adsorbent, the adsorption capability can be restored, and the second adsorbent can be regenerated. This also makes it possible to collect the fluorocarbon A. The fluorocarbon A can be desorbed by, for example, depressurization, heating, the flow of air and nitrogen, or the flow of heated air and heated nitrogen.

The pressure (gauge pressure) at the time of the desorption can be preferably 0.1 MPaG or lower, more preferably 0 MPaG or lower, and still more preferably - 0.1 MPaG or lower. The temperature at the time of the desorption can be preferably 15°C to 200°C, more preferably 50°C to 180°C, and still more preferably 100°C to 150°C.

### (Contact between third mixture and second adsorbent)

The separation method of the present disclosure may further comprise bringing the separated third mixture and the second adsorbent into contact with each other. Such an operation makes it possible to obtain a new mixture having a further reduced concentration of the fluorocarbon A.

The number of times of the contact between the second adsorbent and the mixture is not limited, but can be preferably 1 to 5 and more preferably 1 to 3. The second adsorbents that are used in two or more contacts may be the same as or different from one another.

The operation and conditions for bringing the mixture obtained by the (m-1)^{th} contact and the adsorbent into contact with each other can be performed in the same manner as the operation and conditions for bringing the first mixture and the adsorbent into contact with each other.

### (Purification)

The separation method of the present disclosure may further comprise purifying the separated third mixture. Such purification may comprise obtaining a new mixture having a reduced concentration of the fluorocarbon A as compared with the second mixture.

The number of times of the purification is not limited, but can be preferably 1 to 5 and more preferably 1 to 3. In the present disclosure, the n^{th} purification means an operation for purifying the (n-1)^{th} new mixture obtained by the (n-1)^{th} purification (wherein n is an integer of 2 or more).

In one embodiment, the purification can be performed by distillation or rectification. Such distillation may be performed using a distillation tower generally used, for example, a packed tower or a tray tower. A purified product is distilled as a distillate from the top portion of the distillation tower, and the residue is distilled as a bottom from the bottom portion of the distillation tower. The number of theoretical plates of the distillation tower may be, for example, 1 to 100 plates. The pressure (gauge pressure) at the time of the distillation or rectification may be, 0 MPaG or higher and 5 MPaG or lower. The temperature of the top portion of the distillation tower may be, for example, -60°C or higher and 100°C or lower, and the temperature of the bottom portion of the distillation tower may be, for example, 50°C or higher and 200°C or lower. During the distillation, an extraction solvent may coexist.

The rectification can be performed by performing an operation of distilling the distillate again (operation of performing distillation twice or more).

In the present embodiment, the third mixture is purified, but the present disclosure is not limited thereto. For example, the first mixture may be purified before the first mixture and the adsorbent are brought into contact with each other. That is, a mixture purified by the above operation may be brought into contact with the adsorbent.

### (System)

A fluorocarbon purification system that performs the second separation method is also included in the technical scope of the present disclosure.

The system preferably comprises
an adsorbent, and
the adsorbent is capable of separating, from a first mixture comprising a fluorocarbon A having n carbon atoms and one or more double bonds and a fluorocarbon B having m carbon atoms and no double bonds,
a third mixture in which the composition ratio between the fluorocarbon A and the fluorocarbon B is different from that in the first mixture.

As one example of the system in the present embodiment, a two-stage type system is schematically shown in Figure 2, which will now be described.

In the system of Figure 2, the first mixture is stored in a gas tank 1. The first mixture is supplied to a first-stage separation module 4c through a pressure regulator 2a and a mass flow controller 3a. In one embodiment, the back pressure of the first mixture that is supplied to the separation module 4c can be controlled under increased pressure with the pressure regulator 2a, but is not limited to such an embodiment. The pressure regulator 2a may not be provided.

The separation module 4c comprises an adsorbent 11a, and the first mixture is supplied to such an adsorbent 11a.

In a two-stage type separation instrument, after the contact with the adsorbent 11a, the flow rate of the third mixture is measured with the mass flow meter 5, and the third mixture is supplied to a second-stage separation module 4b through a pressure regulator 2b and a mass flow controller 3b. In one embodiment, the back pressure of the third mixture that is supplied to the second-stage separation module 4b can be controlled under increased pressure with the pressure regulator 2b, but is not limited to such an embodiment. The pressure regulator 2b may not be provided.

The second-stage separation module 4d comprises an adsorbent 11b, and the third mixture is supplied to such an adsorbent 11b.

A new mixture separated by the contact between the third mixture and the adsorbent 11b is passed through a pressure regulator 2c, the gas composition is analyzed with a gas composition analyzer 8b, and then the new mixture is collected to a collection pipe 9. In one embodiment, the flow rate of the new mixture separated by the contact with the adsorbent 11b may be measured using a mass flow meter. In one embodiment, a gas composition analyzer 8a may not be provided.

Although the two-stage separation instrument has been described in the present embodiment, the instrument is not limited thereto, and the instrument may be can be appropriately modified, and for example, a one-stage, three-stage or four or more-stage separation instrument.

In the case of a p-stage type (p is 3 or more), in a (p-1)^{th}-stage separation module, the (p-1)^{th} mixture separated by the contact with an adsorbent may be supplied to an separation module through a pressure regulator and a mass flow controller and may be brought into contact with an adsorbent that is comprised in the separation module to separate further a p^{th} new mixture. p^{th}-stage separation can be performed by collecting the p^{th} mixture after gas composition analysis that can be performed as necessary.

### (Third embodiment: Composite material)

A composite material comprising a metal-organic framework and the fluorocarbon A is also included in the technical scope of the present disclosure. The metal-organic framework and the fluorocarbon A are the same meanings as the metal-organic framework and the fluorocarbon A in the first embodiment.

In such a composite material, the metal-organic framework preferably comprises a metal ion and one or more organic ligands. The metal ion is one or more selected from a Fe ion, a Cu ion, a Ni ion, a Mg ion, a Co ion, a Cr ion, a Zr ion, and a Zn ion, and preferably one or more selected from Fe²⁺, Fe³⁺, Cu²⁺, Ni²⁺, Mg²⁺, Co²+, Cr³⁺, and Zn²⁺. The organic ligand is preferably selected from 1,4-benzenedicarboxylic acid, 1,2-benzenedicarboxylic acid, maleic acid, 1,3,5-benzenetricarboxylic acid, 4,4',4''-(1,3,5-benzenetriyl)trisbenzoic acid, 2,5-dihydroxy-1,4-benzenedicarboxylic acid, oxalic acid, 4,4'-dihydroxy-3,3'-biphenyldicarboxylic acid, 2-hydroxy-1,2,3-propanetricarboxylic acid, 4,4'-bipyridine, triazole, imidazole, 3,3'-bipyrazole, benzimidazole, and 3,5-pyridinedicarboxylic acid and more preferably selected from 1,4-benzenedicarboxylic acid, 4,4'-dihydroxy-3,3'-biphenyldicarboxylic acid, 2-hydroxy-1,2,3-propanetricarboxylic acid, 1,3,5-benzenetricarboxylic acid, and 2,5-dihydroxy-1,4-benzenedicarboxylic acid.

The metal-organic framework preferably comprises an open metal site.

The content of the fluorocarbon A in the composite material is, for example, 1 part by mass or more and 100 parts by mass or less and preferably 10 parts by mass or more and 50 parts by mass or less, per 100 parts by mass of the metal-organic framework.

The composite material may comprise a resin, in addition to the fluorocarbon and a porous organic salt. Examples of such a resin comprise an acrylic resin, a polyurethane resin, a polyolefin resin, a polyester resin, a polyamide resin, a vinyl chloride resin, a styrene resin, a vinyl ether resin, a polyvinyl alcohol resin, a polycarbonate resin, and a polysulfone resin.

The composite material may comprise an additive such as an emulsifier, an antifoaming agent, a surfactant, a leveling agent, a thickener, a viscoelastic modifier, an antifoaming agent, a wetting agent, a dispersant, a preservative, a plasticizer, a penetrant, a fragrance, a bactericide, an acaricide, a fungicide, an ultraviolet absorber, an antioxidant, an antistatic agent, a flame retarder, a dye or a pigment.

### Examples

The present invention will be more specifically described by way of examples below, but the present invention is not limited thereto.

### Example 1 and Comparative Example 1

As adsorbents, HKUST-1 (manufactured by Atomis Inc.) and CALF-20 (manufactured by Atomis Inc.) shown in Table 1 were used. The adsorbents to be used were vacuum-treated at 130°C for 2 h, which served as a pretreatment.

### (Measurement of adsorption breakthroughs of gas mixtures)

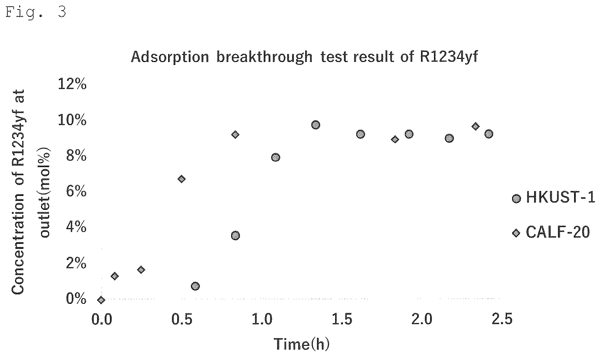

A gas mixture of R1234yf/R134a = 10 mol%/90 mol% was circulated into an adsorption tower packed with the adsorbent in Table 1 at a total pressure of 0.4 MPaG, a temperature of 298 K and a linear velocity of 0.4 cm/second, and the concentrations of R1234yf at the outlet of the adsorption tower was measured. Adsorption breakthrough curves that show the concentration changes of R1234yf are shown in Figure 3. The amounts of R1234yf adsorbed that were found from the adsorption breakthrough curves (the amounts per gram of the adsorbent) are shown in Table 1 as the amounts of gas A adsorbed.

**[Table 1]**

| | Adsorbent | Kind | Metal | Pore diameter (nm) | Gas A | Gas B | adsorption amount (g/g) |
|---|---|---|---|---|---|---|---|
| Example 1 | HKUST-1 | Metal-organic framework | Cu | 1.32 | R1234yf | R134a | 0.084 |
| Comparative Example 1 | CALF-20 | Metal-organic framework | Zn | 0.44 | R1234yf | R134a | 0.004 |

### Reference Signs List

1 Gas tank
2a, 2b, 2c Pressure regulator
3a, 3b Mass flow controller
4a, 4b, 4c, 4d Separation module
5 Mass flow meter
7 Vacuum booster
8a, 8b Gas composition analyzer
9 Collection pipe
10a, 10b Adsorbent comprising metal-organic framework
11a, 11b Adsorbent

## Claims

1. A method for separating a fluorocarbon, comprising:
separating, from a first mixture comprising a fluorocarbon A having n carbon atoms and one or more double bonds and a fluorocarbon B having m carbon atoms and no double bonds,
a second mixture in which a composition ratio between the fluorocarbon A and the fluorocarbon B is different from that in the first mixture,
wherein the separation is performed by bringing the first mixture and an adsorbent comprising a metal-organic framework into contact with each other.

2. The method according to claim 1, wherein the first mixture and the adsorbent comprising a metal-organic framework are brought into contact with each other at a temperature of -10°C or higher and 80°C or lower.

3. The method according to claim 1 or 2, wherein n is 2 or more and 3 or less.

4. The method according to any one of claims 1 to 3, wherein m is 1 or more and 3 or less.

5. The method according to any one of claims 1 to 4, wherein the fluorocarbon A comprises R1234yf.

6. The method according to any one of claims 1 to 5, wherein the fluorocarbon B comprises one or more selected from R134a, R143, R143a, R32 and R125.

7. The method according to any one of claims 1 to 6, wherein the metal-organic framework comprises a metal ion.

8. The method according to any one of claims 1 to 7, wherein the metal-organic framework comprises one or more organic ligands, and the organic ligand comprises two or more groups capable of forming a coordinate bond to a metal ion, per molecule.

9. The method according to any one of claims 1 to 8, wherein the metal-organic framework comprises a metal ion and one or more organic ligands, and the organic ligand comprises two or more groups capable of forming a coordinate bond to the metal ion, per molecule.

10. The method according to any one of claims 1 to 9, wherein the metal-organic framework comprises an open metal site provided by a coordinatively unsaturated metal ion.

11. The method according to any one of claims 1 to 10, wherein the adsorbent comprising the metal-organic framework further comprises a resin.

12. The method according to any one of claims 1 to 11, wherein the adsorbent comprising the metal-organic framework is in a form of powder, granules, flakes, or pellets.

13. The method according to any one of claims 1 to 12, wherein the first mixture is an azeotropic mixture or pseudoazeotropic mixture of fluorocarbons.

14. The method according to any one of claims 1 to 13, further comprising: bringing the separated second mixture and the adsorbent comprising the metal-organic framework into contact with each other.

15. The method according to any one of claims 1 to 14, further comprising: purifying the separated second mixture.

16. The method according to any one of claims 1 to 15, further comprising, after bringing the first mixture and the adsorbent comprising the metal-organic framework into contact with each other:
desorbing the fluorocarbon A adsorbed to the adsorbent comprising the metal-organic framework from the adsorbent comprising the metal-organic framework.

17. A fluorocarbon purification system that performs the method according to any one of claims 1 to 16.

18. A method for separating a fluorocarbon, comprising:
separating, from a first mixture comprising a fluorocarbon A having n carbon atoms and one or more double bonds and a fluorocarbon B having m carbon atoms and no double bonds,
a third mixture having a reduced concentration of the fluorocarbon A as compared with the first mixture,
wherein the separation is performed by bringing the first mixture and an adsorbent into contact with each other.

19. The method according to claim 18, wherein the first mixture and the adsorbent are brought into contact with each other at a temperature of -10°C or higher and 80°C or lower.

20. The method according to claim 18 or 19, wherein n is 2 or more and 3 or less.

21. The method according to any one of claims 18 to 20, wherein m is 1 or more and 3 or less.

22. The method according to any one of claims 18 to 21, wherein the fluorocarbon A comprises R1234yf.

23. The method according to any one of claims 18 to 22, wherein the fluorocarbon B comprises one or more selected from R134a, R143, R143a, R32 and R125.

24. The method according to any one of claims 18 to 23, wherein the adsorbent comprises a porous body.

25. The method according to any one of claims 18 to 24, wherein the adsorbent comprises a metal-organic framework.

26. The method according to claim 25, wherein the metal-organic framework comprises a metal ion.

27. The method according to claim 25 or 26, wherein the metal-organic framework comprises one or more organic ligands, and the organic ligand comprises two or more groups capable of forming a coordinate bond to a metal ion, per molecule.

28. The method according to any one of claims 25 to 27, wherein the metal-organic framework comprises a metal ion and one or more organic ligands, and the organic ligand comprises two or more groups capable of forming a coordinate bond to the metal ion, per molecule.

29. The method according to any one of claims 25 to 28, wherein the metal-organic framework comprises an open metal site provided by a coordinatively unsaturated metal ion.

30. The method according to any one of claims 18 to 29, wherein the adsorbent further comprises a resin.

31. The method according to any one of claims 18 to 30, wherein the adsorbent is in a form of powder, granules, flakes, or pellets.

32. The method according to any one of claims 18 to 31, wherein the first mixture is an azeotropic mixture or pseudoazeotropic mixture of fluorocarbons.

33. The method according to any one of claims 18 to 32, further comprising: bringing the separated third mixture and an adsorbent into contact with each other.

34. The method according to any one of claims 18 to 33, further comprising: purifying the separated third mixture.

35. The method according to any one of claims 18 to 34, further comprising, after bringing the first mixture and the adsorbent into contact with each other:
desorbing the fluorocarbon A adsorbed to the adsorbent from the adsorbent.

36. A fluorocarbon purification system that performs the separation method according to any one of claims 18 to 35.

37. A composite material comprising:
a metal-organic framework; and
a fluorocarbon A having n carbon atoms and one or more double bonds,
wherein the metal-organic framework comprises an open metal site provided by a coordinatively unsaturated metal ion.
